# EUROPEAN PATENT SPECIFICATION

(11) **EP 0 386 246 B1**
(45) Date of publication and mention of the grant of the patent: **16.10.1996**
(21) Application number: 89902542.3
(22) Date of filing: 17.02.1989
(51) Int. Cl.: A61N 5/02

(54) **DEVICE FOR LOCAL HEATING AND STEREORESONATOR FOR LOCAL HEATING**
ANORDNUNG ZUR LOKALEN HEIZUNG UND STEREORESONATOR DAZU
DISPOSITIF D'ECHAUFFEMENT LOCAL ET STEREORESONATEUR POUR L'ECHAUFFEMENT LOCAL

(30) Priority: 18.02.1988 JP 34049/88
(43) Date of publication of application: 12.09.1990
(73) Proprietor: OMRON CORPORATION, Kyoto-shi, Kyoto-fu 617 (JP); SAITO, Yoshiaki, Niigata 950-21 (JP); MATSUDA, Jinichi, Nigata 940 (JP); KATO, Kazuo, Kariwa-gun Niigata 945-02 (JP)
(72) Inventor: MATSUDA, Jinichi, Niigata 940 (JP); KATO, Kazuo, Kariwa-gun Niigata 945-02 (JP)
(74) Representative: Monnier, Guy
(86) International application number: PCT/JP89/00163
(87) International publication number: WO 89/07469

(56) References cited:
- EP-A- 0 034 735
- EP-A- 0 171 620
- JP-A-61 209 672
- JP-Y- 0 604 516
- JP-Y- 0 622 060
- US-A- 4 378 806

## Description

### Technical Field

The present invention relates to a heating apparatus which can heat a body to be heated satisfactorily up to the deep portion thereof and heat a desired portion intensively, and specifically relates to a locally heating apparatus which supplies high-frequency energy to a stereo resonator such as a cavity resonator, and heats a local portion by electromagnetic field generated by that energy.

### Technical Background

Local heating which heats a specific portion has been performed extensively in various technical fields. for example, in the field of medical treatment, treatments of blood circulation disorder, inflammation disease, neuralgia and the like are performed by applying high-frequency energy to an affected portion of a human body. Also, recently, it is known that cancer cells perish by heating cancer tissue at 43°C or more, and therefore the medical treatment of cancer which locally heats the cancer tissue portion has been performed.

Fig. 20 shows a basic configuration of a conventional locally heating apparatus which heats a human body for the purpose of treatment of cancer.

A pair of plane electrodes 91 and 92 are installed in a manner of sandwiching an affected portion 81. When high-frequency energy is applied to a pair of the plane electrodes 91 and 92 from a high-frequency power supply unit 93, the affected portion 81 is heated between the plane electrodes 91 and 92.

In this case, a fat layer exists on the surface of the human body, and this fat layer is particularly easy to be heated since it is positioned beneath the electrodes, and therefore cooling parts 94 and 95 are installed beneath the plane electrodes 91 and 92, and cooling water is circulated by a cooling apparatus 96. Thereby, the human body is prevented from suffering and adverse effect by unnecessary heating of the fat layer.

In the conventional locally heating apparatus using a pair of plane electrodes, heating is performed by a current, and therefore it has tendency that the tissue having a high electric resistance like the fat layer on the surface of the human body is heated more intensely than the tissue having a low electric resistance, and a deeper part of the human body apart from the electrode is harder to be heated. For this reason, there exist problems such that heating concentrated on a local portion is difficult to be performed, and a local scald can be caused in the normal tissue of surface fat portion or the like by a long period heating.

For example, when the cancer tissue is heated at 43°C or higher, cells of cancer perish, but when the cells of cancer existing in the deep part of the human body are intended to be heated at 43°C or higher, in the conventional locally heating apparatus, there is a risk that the tissue close to the plane electrode is heated at 45°C or higher which is the limit temperature thereof, and therefore, the cells of cancer located in the deep part cannot be heated satisfactorily. For this reason, in the conventional locally heating apparatus, it is the present state that only the cancer existing near the surface of the human body can be treated.

US-A-4.378.806 discloses a local heating apparatus using a microwave beam. Said apparatus includes a microwave source, a resonant cavity beam divider, wave guides for guiding the divided beam and matching media provided at each end of the waveguides. A local portion of the body is held by the matching media and the local portion is heated by the microwave beam. The matching media matches an intrinsic impedance of the dielectric load to the intrinsic impedance of any medium within the waveguides. Unnecessary heat is removed from dielectric fluid of a dielectric matching media by circulating the dielectric fluid to a heat exchanger.

EP-A-0.171.620 discloses a microwave-heating apparatus for making enzymes in cells of a brain of an animal to be tested inactivated in a moment using the microwave. According to this document, the microwave-heating apparatus includes a high-power microwave oscillator, a waveguide connected to the oscillator, an impedance matching part connected to the output part of the waveguide, an applicator and a container for fixing a head of the animal. According to EP-A-0.171.620, since the rise time of the ouput power of the oscillator is shorter than the time to apply the microwave to the testing animal and heating to each testing animal is performed so efficiently by matching the impedance without influencing any effect on the testing animal before heating, the animal can be heated very efficiently hereby to obtain respectable data.

A resonant cavity disclosed in US-A-4.378.806 corresponds to a local heating apparatus using a pair of plane electrodes described in the background art of the present application. Therefore, it is difficult to heat a deeper part of the human body apart from the electrode and it is possible that a local scald can occur in the normal tissue if a locally concentrated heating is effected. Therefore, it is necessary to remove heat by using a heat exchanger according to US-A-4.378.806.

According to EP-A-0.171.620, it is possible to heat the head of the animal under the same heating condition, the object to be heated is only a part of a small animal and it is not possible to apply the same to the human body.

Therefore, the object of the present invention is to provide a local heating apparatus capable of handling the human body which should be treated carefully, heating a deeper part of the human body well and heating the desired local portion by removing shortcomings of the conventional local heating apparatus.

Accordingly, the present invention purposes to provide a locally heating apparatus which has not the deficiency of the conventional locally heating apparatus, and can heat a body to be heated satisfactorily up to the deep part and can intensively heat a desired local portion and a stereo resonator for local heating suitably for this apparatus.

### Disclosure of the Invention.

That the conventional locally heating apparatus cannot heat satisfactorily up to the deep part of the human body is caused by that heating is made by a current and the current diffuses as it parts from the electrode.

The present invention as defined by Claims 1 and 13 aims at this point, and confines the electric field or the current inside by the action of the magnetic field existing in the periphery thereof to prevent diffusion of the current.

To generate a concentrated electric field for heating by confining an electric field or a current, the cavity resonator for local heating of the present invention has a basic configuration such that a cavity is formed, said cavity being of cylindrical shape, in whose axis a conductive cylinder of smaller diameter is arranged to form an inner conductor, an electromagnetic field being generated in the gap between the face of the inner conductor and the cavity by a source of microwave energy coupled to the cavity by a probe.

When high frequency energy is applied to the cavity resonator having this configuration and a resonance is generated in the cavity, a strong electric field is generated by the action of the inner electrode between the inner electrode and the conductor of the cavity, or between two inner electrodes where they are disposed in an opposite fashion, and a strong magnetic field is generated so as to surround the electric field.

This strong magnetic field confines the electric field to prevent diffusion thereof, and generates a strong field concentrating on the center axis of the inner electrode.

Thus, heating can be performed satisfactorily up to the deep part of the body to be heated.

Also, the locally heating apparatus of the present invention adopts a basic configuration that the above-mentioned stereo resonator is provided with a high-frequency power supply unit.

By this configuration, heating can be performed satisfactorily up to the deep part of the body to be heated, and a desired local portion can be heated intensively without scalding the normal tissue of the surface.

### Brief Description of the Drawings

Fig. 1 is an explanatory view of a basic configuration of a stereo resonator of the present invention and a configuration of an embodiment 1 of the same.

Fig. 2 is an explanatory view of a configuration of an embodiment 2 of the same stereo resonator.

Fig. 3 is an explanatory view of a configuration of an embodiment 3 of the same stereo resonator.

Fig. 4 is an explanatory view of a configuration of an embodiment 4 of the same stereo resonator.

Fig. 5 is an explanatory view of a configuration of an embodiment 5 of the same stereo resonator.

Fig. 6 is an explanatory view of a configuration of an embodiment 6 of the same stereo resonator.

Fig. 7 is an explanatory view of a configuration of an embodiment 7 of the same stereo resonator.

Fig. 8 is an explanatory view of a configuration of an embodiment 8 of the same stereo resonator.

Fig. 9 is an explanatory view of a configuration of an embodiment 9 of the same stereo resonator.

Fig. 10 is an explanatory view of a configuration of an embodiment 10 of the same stereo resonator.

Fig. 11 is an explanatory view of a configuration of an embodiment 11 of the same stereo resonator.

Fig. 12 is an explanatory view of a basic configuration of a locally heating apparatus of the present invention and a configuration of an embodiment 1 of the same.

Fig. 13 is an explanatory view of an embodiment 2 of a configuration of the same locally heating apparatus.

Fig. 14 is an explanatory view of a configuration of an embodiment 4 of the same locally heating apparatus.

Fig. 15 is an explanatory view of a configuration of an embodiment 5 of the same locally heating apparatus.

Fig. 16 is an explanatory view of a configuration of an embodiment 6 of the same locally heating apparatus.

Fig. 17 is an explanatory view of a configuration of an embodiment 7 of the same locally heating apparatus.

Fig. 18 is an explanatory view of a configuration of an embodiment 8 of the same locally heating apparatus.

Fig. 19 is an explanatory view of results of heating experiment by the locally heating apparatus of the present invention.

Fig. 20 is an explanatory view of a conventional locally heating apparatus.

### Best Form for Embodying the Present Invention

To describe the present invention in further detail, hereinafter description is made thereon according to the appended drawings.

In addition, for the sake of convenience in description, embodiments of the stereo resonator for local heating and embodiments of the locally heating apparatus of the present invention are described separately.

### (A) Embodiments of Stereo Resonator

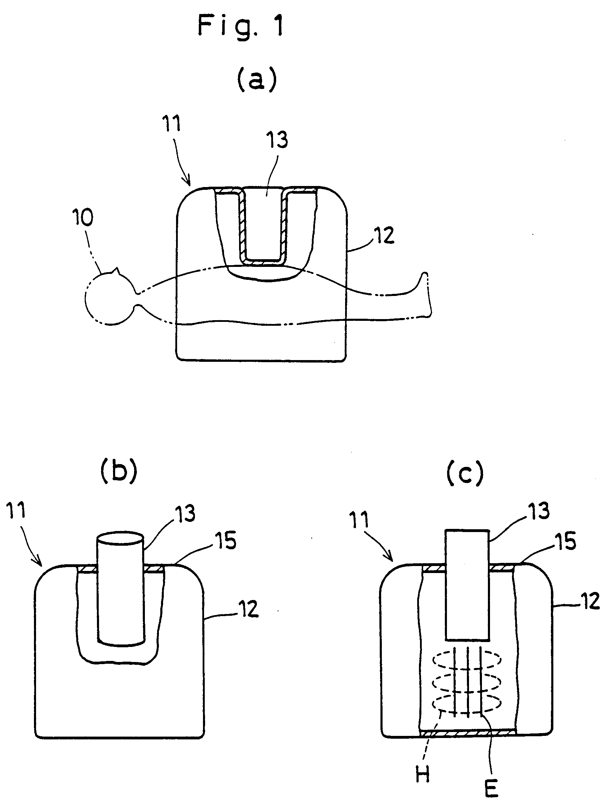

Description is made on embodiments of the stereo resonator in accordance with the present invention in reference to Fig. 1 through Fig. 11. In addition, description on the aspect of an electric field formed by inner electrodes 13 of each stereo resonator 11 of the present invention is made in the item of heating experiment. Also, on the drawing of each embodiment, illustration of configurations not directly relating to the configuration of the embodiment is omitted unless required.

### (A1) Embodiment 1

A stereo resonator 11 is constituted in a manner that an inner electrode 13 is formed by hollowing part of a cavity 12 constituted with a conductor as shown in Fig. 1 (a) or by a conductor such as a separate conductor cylinder or a conductor rod as shown in Fig. 1 (b).

A body 10 to be heated is carried in between the inner electrode 13 and the opposite conductor of the cavity 12. The cross-section of the inner electrode 13 can be designed in various shapes such as circular, elliptic and rectangular shapes.

When high-frequency energy is supplied to the stereo resonator 11 from exterior and a resonance is generated in the cavity 12 by this configuration, a strong electric filed E is generated between the inner electrode 13 and the opposite conductor of the cavity 12 as shown in Fig. 1 (c), and a strong magnetic field H is generated in a manner of surrounding the electric field E. This strong magnetic field H confines the electric field E so as not to diffuse, and generates a strong electric field concentrating on the center axis of the inner electrode 13. In addition, detailed description is made on the aspect of this electric field in the item of heating experiment of the locally heating apparatus.

By this configuration, heating can be performed satisfactorily up to the deep part of the body 10 to be heated. Also, a desired local portion the normal tissue of the surface.

### (A2) Embodiment 2

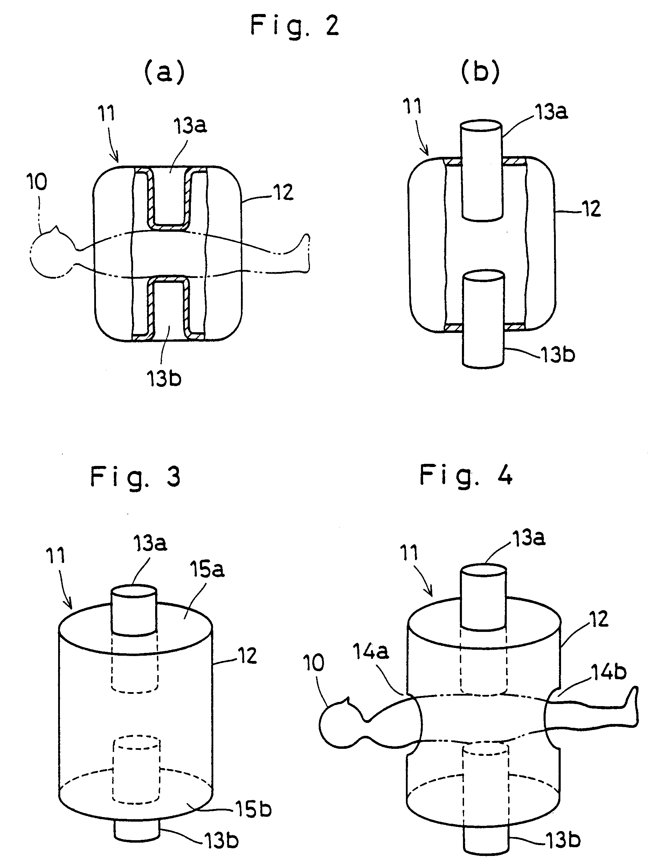

Two inner electrodes 13a and 13b are installed in a manner of facing each other. The body 10 to be heated is carried in between the both inner electrodes 13a and 13b. Fig. 2(a) shows an example of constituting the inner electrodes 13a and 13b by hollowing part of the conductor of the cavity 12, and Fig. 2(b) shows an example of constituting the inner electrodes 13a and 13b with separate conductors such as conductor cylinders or conductor rods. In addition, it is also possible that one of the inner electrodes 13a and 13b is constituted with a hollow and the other is constituted with a conductor.

The body 10 to be heated is carried in between the both inner electrodes 13a and 13b. This configuration can raise the degree of concentration of the electric field and improve the heating effect.

Also, when the human body is heated using the stereo resonator 11, as described later in the item of heating experiment, a new effect is obtained that heating of the tissue having a high electric resistance such as the fat layer and bones not wanted to be heated originally is suppressed at a low degree, and the cancer tissue which is the target of treatment is heated effectively and the effect of treatment is improved to a great extent. Also, since the diameter of the portion to be heated is reduced to a small value, the risk of heating the normal tissue is decreased to a great extent in comparison with the conventional technology. Thus, this configuration has an effect capable of solving the problem peculiar to heating the human body.

### (A3) Embodiment 3

The cavity 12 is constituted with a cylindrical conductor as shown in Fig. 3, and the inner electrodes 13 (13a and 13b) are installed on the end surfaces thereof 15 (15a and 15b). For the stereo resonator constituted with the cavity 12 of this cylindrical conductor, for example, there is the reentrant-type resonator. Fig. 3 shows an embodiment wherein the inner electrodes 13a and 13b are constituted with two conductors facing each other. By installing the inner electrodes 13 on the end surfaces 15 in such a manner, a nice state of resonance is obtained, and the intensity and concentration of the electric field are intensified, and thereby the heating effect can be improved.

### (A4) Embodiment 4

As shown in Fig. 4, entrances 14 (14a and 14b) for carrying in/out the body 10 to be heated are installed in the cavity 12 to enable the body 10 to be heated to be easily carried into the cavity 12. Where the whole of the body 10 to be heated is put in the cavity 10, one entrance 14 will do. In this case, when an open-close lid constituted with a conductor is attached to the entrance (not illustrated), the electromagnetic field is prevented from leaking outside the cavity at heating, and therefore the safety can be secured. Also, since the high-frequency current flows also, through the open-close lid, the electromagnetic field in the cavity 12 is not disturbed, and a nice state of resonance can be realized.

Thus, by installing the entrances 14 in the cavity 12, the body 10 to be heated can be easily carried in and out, and the workability is improved.

### (A5) Embodiment 5

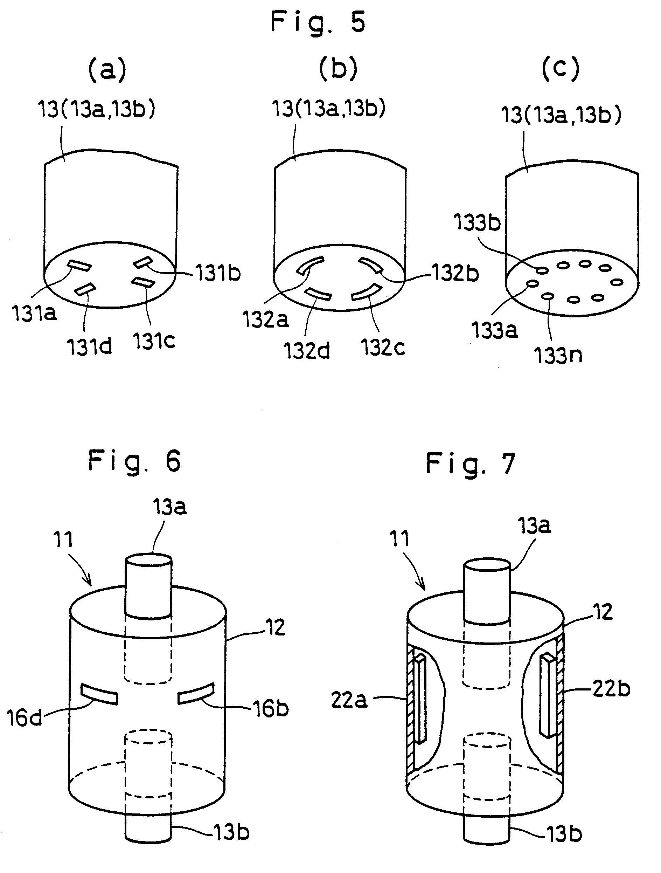

When the electrode surface of the inner electrode 13 is formed in a structure of variable distribution of electric field as shown in Fig. 5, the concentration of the electric field is alleviated and the heating region can be widened.

The structure of variable distribution of electric field can be realized, for example, by installing slits or recesses or protrusions 131a - 131d on the electrode surface in the radial direction as shown in Fig. 5(a), by installing slits or recesses or protrusions 132a - 132d on the electrode surface in the circumferential direction as shown in Fig. 5(b), or by installing small holes 133a - 133n on the electrode surface in the circumferential direction as shown in Fig. 5(c).

The shape, position, number and the like of these slits, recesses, protrusions or holes are selected properly in correspondence to the range of the heating region.

Also, the structure of variable distribution of electric field can be realized by the following methods.
1 The diameter of the inner electrode 13, particularly the diameter of the tip electrode portion thereof is made larger.
2 The shape of the inner electrode 13, particularly the shape of the tip electrode portion thereof is formed to match with the range of the heating region (for example, ellipse).
3 The tip electrode portion of the inner electrode 13 is formed in a structure of diving it into a plurality of pieces.

### (None of them is illustrated.)

In addition, it is also possible that the tip electrode portion of the inner electrode is designed in a structure of removable adaptor in advance, the tip electrode adaptors having the above-described structures of variable distribution of electric field are prepared respectively, and a tip electrode adaptor suitable for the range of the heating region is attached. Also, where two or more inner electrodes (13a and 13b) are installed, it is also effective that at least one of them is formed in a structure of variable distribution of electric field.

Thus, the structure of variable electric field makes it possible to adjust the range of concentration of electric field, and can expand the heating region corresponding to the range of the portion to be heated. For example, when the diameter of the cancer tissue is as small as several cm or less, heating can be performed nicely by a combination of the stereo resonator 11 and the inner electrodes 13. However, for a larger cancer tissue, the whole of the large cancer tissue can be heated nicely by expanding the range of concentration of electric field by means of such a structure of variable distribution of electric field.

### (A6) Embodiment 6

Adjustment of the range of concentration of the electric field formed by the inner electrodes 13 (13a and 13b), as shown in Fig. 6, can be realized by installing electric field distribution adjusting holes 16 (16a and 16b) in part of the conductor constituting the cavity 12. This means that by installing these electric field distribution adjusting holes 16 (16a and 16b), the whole distribution of electromagnetic field in the cavity 12 is changed, and attending on this change, the range of concentration of the electric field formed by the inner electrodes 13 (13a and 13b) is changed.

As shown in Fig. 6, the electric field distribution adjusting holes 16 (16a and 16b) work effectively when they are installed in a slit shape in the direction orthogonal to the direction of high-frequency current flowing through the conductor constituting the cavity 12, and they may be simple small holes. The number, shape and positions of installation of the electric field distribution adjust holes are selected properly in correspondence to the range of the heating region.

In this case, by attaching sliding-type open-close lids (not illustrated) to the electric field distribution adjusting holes 16 (16a and 16b) and making the size of the holes variable, the range of concentration of electric field can be made variable.

Thus, by installing the electric field distribution adjusting holes 16 in part of the cavity 12, the range of concentration of electric field can be expanded and heating can be performed nicely over a wide range like the case of the embodiment 5.

### (A7) Embodiment 7

As shown in Fig. 7, adjustment of the range of concentration of the electric field formed by the inner electrodes 13 (13a and 13b) is realized by installing electric field adjusting members 22 (22a and 22b) in the cavity 12.

The electric field adjusting members 22 (22a and 22b) are constituted with conductors, dielectrics or the like, and work effectively when installed at places of a high HF electric field level in the cavity 12. These electric field adjusting members 22 (22a and 22b) change the whole distribution of electromagnetic field the cavity 12, and attending on this change, the rage of concentration of the electric field formed by the inner electrodes 13 (13a and 13b) is also changed.

The shape, number, positions of mounting of the electric field adjusting members 22 (22a and 22b) are selected properly in correspondence to the range of the heating region.

### (A8) Embodiment 8

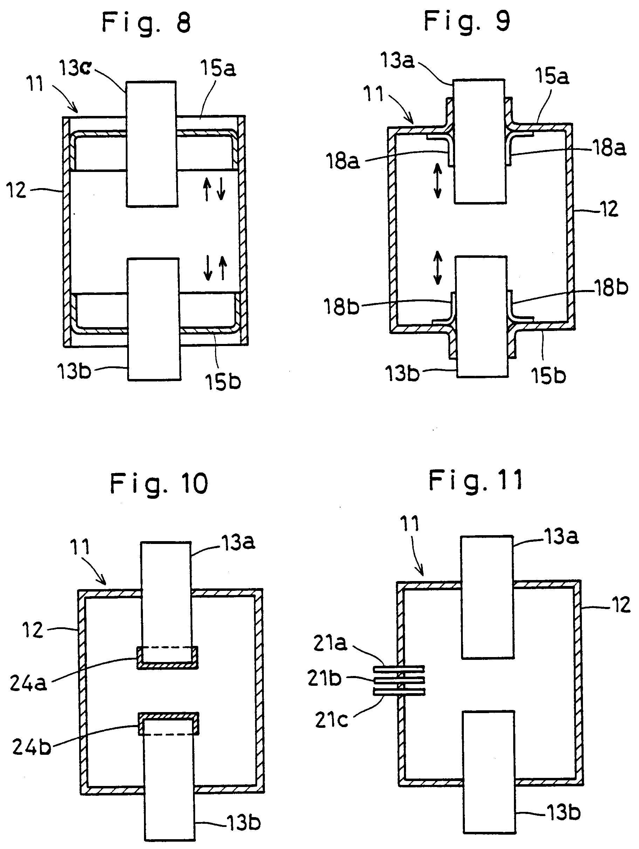

The resonance frequency of the stereo resonator 11 varies depending on the volume and shape of the cavity. Accordingly, as shown in Fig. 8, this can be realized by making part of the conductor constituting the cavity 12 movable.

In Fig. 8, the top and bottom end surfaces 15a and 15b of the cylindrical cavity 12 are both made movable. In this case, only one of the end surfaces 15a and 15b may be made movable. Where the cavity 12 is rectangular, the side surface may be made movable. This configuration makes it possible to adjust the resonance frequency of the stereo resonator 11 and put the resonance in the best condition. Also, in reverse, the output, that is, heating ability can be adjusted by shifting the resonance frequency from the frequency of the best resonance condition.

### (A9) Embodiment 9

The thickness of the body 10 to be heated varies depending on each body to be heated, and it is preferable to bring the electrode surface of the inner electrode 13 and the body 10 to be heated in the best state of contact with each other all the time.

This is realized by adopting a structure of variable position of the electrode surface of at least one of the inner electrodes 13 constituted with a conductors such as a conductor cylinder or conductor rod.

Fig. 9 shows an example of the variable structure of the position of the electrode surface, and the inner electrodes 13a and 13b constituted with a conductor are attached to the end surfaces 15a and 15b in a manner capable of moving up and down.

Up-down movement of the inner electrodes 13a and 13b can be performed by various methods, and in this case, the density of the high-frequency current at the portion of contact of the inner electrodes 13a and 13b with the end surfaces 15a and 15b is large and therefore power loss is generated unless a good contact is kept. To prevent this power loss, contact members 18a and 18b constituted with slidable spring material are attached to the end surfaces 15a and 15b, and the inner electrodes 18a and 18b move up and down while sliding on this contact members 18a and 18b.

In addition, only one of the inner electrodes 13a and 13b may be made movable up and down.

### (A10) Embodiment 10

When the output power of the stereo resonator 11 is large, a discharge might occur from the electrode surface of the inner electrode 13.

As shown in Fig. 10, this discharge can be prevented by covering the electrode surfaces of the inner electrodes 13 (13a and 13b) with insulating parts 24 (24a and 24b).

In addition, this insulating member 24 works effective even when it is installed in either of the inner electrodes 13a and 13b.

Also, a discharge can be prevented by forming space between the body 10 to be heated and the inner electrode 13.

### (A11) Embodiment 11

The resonance frequency realizing the best state of resonance of the stereo resonator 11 is critical, and therefore to realize the best state of resonance, fine adjustment of the resonance frequency is required to be made. In addition, the oscillation deviates from the state of resonance, the output voltage drops, and accordingly the heating ability is also reduced.

As shown in Fig. 11, to make this fine adjustment of the resonance frequency, frequency adjusting members 21 (21a, 21b and 21c) are mounted in the cavity 12 in a manner capable of insertion.

These frequency adjusting members 21a - 21c are constituted with a conductor rod, and the effect thereof is large when they are mounted on the portion where the level of the electric field generated by the HF current flowing through the cavity 12 is high. The number of the frequency adjusting members 21 (21a - 21c) is selected responding to the variable range of frequency required. Also, adjustment of the position of insertion (amount of insertion) is made by various methods, and can be made by automatic control as shown in the following item (B2).

### (B) Embodiments of Locally Heating Apparatus

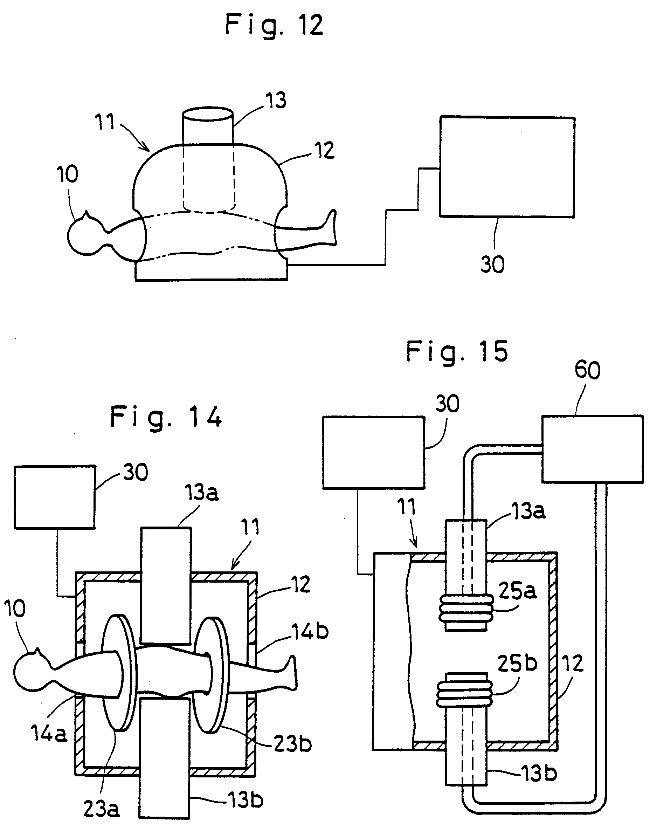

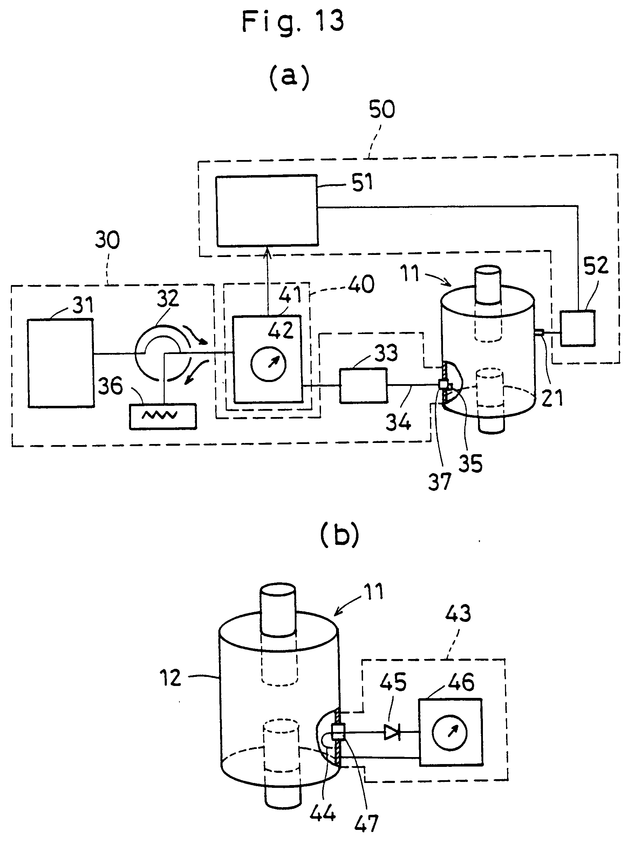

Description is made on embodiments of the locally heating apparatus in accordance with the present invention in reference to Fig. 12 through Fig. 18.

### (B1) Embodiment 1

A locally heating apparatus of the present invention is configurated using each stereo resonator 11 for local heating as exemplified in the above-described items (A1) - (A11).

In Fig. 12, the stereo resonator 11 is any one of the stereo resonators for local heating as described in the above-described items (A1) - (A11). High-frequency energy is supplied from a high-frequency power supply unit 30 to this stereo resonator 11, and puts it in the state of resonance.

In the resonant state of the stereo resonator 11, the inner electrode 13 generates a strong electric field of high concentration, and thereby the body 10 to be heated is heated locally up to a sufficient depth. Also, a desired local portion can be heated intensively without scalding the normal tissue of the surface.

In addition, description on a specific configuration of the high-frequency power supply unit 30 is made together in the following embodiment 2.

### (B2) Embodiment 2

Another locally heating apparatus of the present invention is constituted using the stereo resonator 11 as described in the above-described item (A11).

In Fig. 13, the basic configuration and the operation of the stereo resonator 11, the high-frequency power supply unit 30, the resonant state detecting means, and the position controlling means 50 are as described previously.

In the high-frequency power supply unit 30, high-frequency energy generated from a high-frequency energy generator 31 passes through a directional coupler 32, an impedance matching unit 33 and a cable 34, and is supplied to the stereo generator 11 by coupling it through a coupling probe 35 in the cavity 12.

The impedance matching unit 33 performs impedance matching between the directional coupler 32 and the stereo resonator 11. This matching can be performed also automatically.

The directional coupler 32 delivers the high-frequency energy from the high-frequency energy generator to the stereo resonator 11 side, and delivers the high-frequency energy reflected from the stereo resonator 11 to a resistor 36. Thereby, reflected high-frequency energy can be prevented from entering the high-frequency energy generator and breaking it. A connector 37 is attached to the cavity 12, and connects the cable 34 and the coupling probe 35.

As shown in Fig. 13 (a) the resonant state detecting means 40 can be constituted with a standing wave detector 41. The standing wave detector 41 is coupled between the directional 32 and the impedance matching unit 33, and detects standing wave and indicates it by a standing wave indicator 42.

As shown in Fig. 13 (b), the resonant state detecting means 40 can be constituted with an amplitude detector 43. The amplitude detector 43 takes out high-frequency output of the stereo resonator 11 by a detecting probe 44 installed in the cavity 12, converts it into a DC by a rectifier 45, and thereafter makes an amplitude indicator 46 indicate this DC value. This DC value indicates the amplitude of high-frequency output of the stereo resonator 11. Numeral 47 designates a connector connecting the detecting probe 44 and the rectifier 45 side.

In the position controlling means, numeral 51 designates a detection controlling unit, and numeral 52 designates a driving unit making positional adjustment by driving a frequency adjusting member 21, which is constituted with a motor.

When the resonant state detected by the resonant state detecting means 40 constituted with the standing wave detector 41 and the amplitude detector 43 is inputted to the position controlling means 50, the detection controlling unit 51 directs the driving unit 52 to adjust the amount of insertion of the frequency adjusting member 21 into the cavity 12 in correspondence to this detected resonant state. The driving unit 52 turns the motor clockwise or counter-clockwise according to a given direction to adjust the amount of insertion of the frequency adjusting member 21 into the cavity 12.

In this case, in the standing wave method using the standing wave detector 41, the amount of insertion of the frequency adjusting member 21 is controlled to minimize the standing wave ratio or the reflected wave indicated by the standing wave indicator 42. In the amplitude indicating method using the amplitude detector 43, control is performed so that the amplitude value indicated by the amplitude indicator 46 becomes maximum.

Thus, the stereo resonator 11 can be maintained in the best state of resonance all the time and a nice heating can be continued even if a person moves during treatment or the condition of resonance of the stereo resonator 11 is varied by a change in electric constant due to a rise in the temperature of the body 10 to be heated.

Also, the electromagnetic field distribution in the resonator is sometimes disturbed by a change in the electric constant or the like, resulting in a pain or a local scald of the human body or a breakage of the high-frequency energy generator 31, but these troubles can also be prevented, and a safe and effective heating or treatment can be performed.

### (B3) Embodiment 3

The output power is maximum in the resonant state, and when the frequency of high-frequency energy supplied to the stereo resonator 11 deviates from the resonance frequency, the output power for heating is reduced. Accordingly, the output power for heating of the stereo resonator 11 can be adjusted by varying the frequency of high-frequency energy supplied to the stereo resonator 11.

Varying the frequency of high-frequency energy supplied to the stereo resonator 11 can be easily realized by modifying the high-frequency energy generator 31 of the high-frequency power supply unit 30 into a variable frequency type. Furthermore, where the high-frequency energy generator 31 can vary its output stepwise, by combining this with frequency adjustment, the output of high-frequency energy can be adjusted continuously from a low level to a high level over a wide range.

Accordingly, the output power, that is, the heating ability of the stereo resonator 11 can be adjusted over a wide range.

### (B4) Embodiment 4

When the electric field leaks from the inner electrode 13, portions other than the heated portion of the body 10 to be heated are heated, and a harmful action might occur.

To prevent this trouble, as shown in Fig. 14, shielding members 23 (23a and 23b) are installed in the cavity to cut off leakage of the electromagnetic field to portions other than the heated portion of the body 10 to be heated.

The shielding members 23 (23a and 23b) are constituted with a conductor or a dielectric, and either one of the members 23a and 23b will do for this purpose, and further the members can be increased in number.

### (B5) Embodiment 5

Where the output power of the stereo resonator 11 is large, the inner electrode 13 rises in temperature and generates heat, and when the body 10 to be heated is a human body, a scald might occur.

To prevent this trouble, as shown in Fig. 15, cooling members 25 (25a and 25b) are installed in the inner electrodes 13 (13a and 13b), and cooling water is circulated by a cooling unit 60.

The cooling members 25 (25a and 25b) can be constituted, for example, in a manner that cooling pipes are attached to the outside of the inner electrodes (13a and 13b) in a manner of winding them or they are attached to the insides of the inner electrodes 13. Either one of the cooling members 25 (25a and 25b) will do for an effective operation.

### (B6) Embodiment 6

When the body 10 to be heated is not flat or moves during heating, a gap is formed between the inner electrode and it, resulting in a reduction in the heating ability.

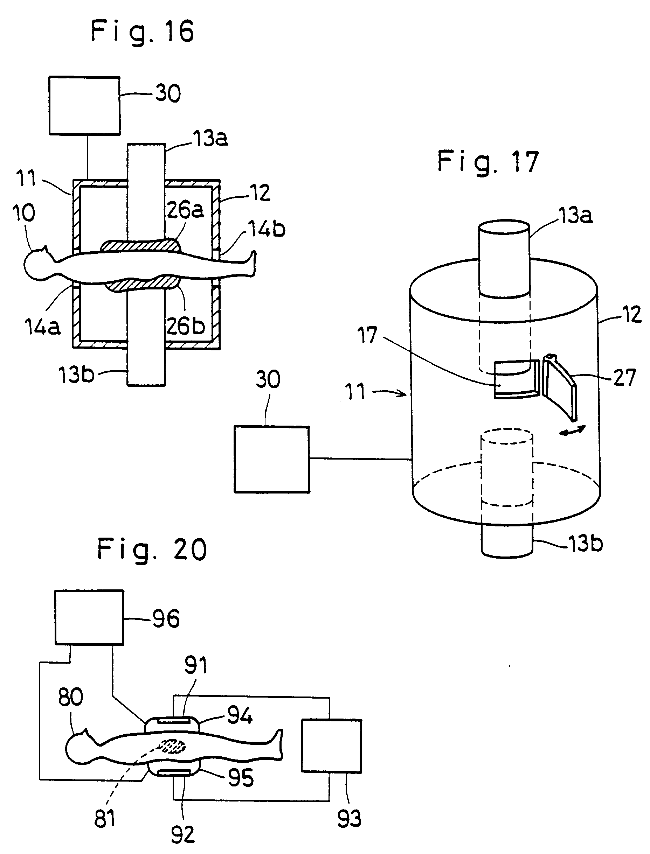

To prevent this reduction, as shown in Fig. 16, low-loss gap members 26 (26a and 26b) are inserted between the inner electrodes 13 (13a and 13b) and the body to be heated.

The gap members 26 (26a and 26b) are constituted, for example, with a bag containing conductor particles, low-pass insulating particles, gas or liquid. Where such a bag is used, even if the body 10 to be heated is not flat-shaped or moves, the bag fills the gaps between the inner electrodes 13 (13a and 13b) and the body 10 to be heated, and can keep the contact of the both in a good state. Even either of these gap members 26a and 26b works effectively.

In addition, this gas or liquid can have a continued cooling effect by passing through pipes connected to an external cooling unit (not illustrated).

### (B7) Embodiment 7

To facilitate attachment and positional adjustment of the gap members 26 (26a and 26b) and check and adjustment of each part in the cavity 12, as shown in Fig. 17, at least one window having a conductive open-close lid 27 is installed in the cavity 12.

When the conductive open-close lid 27 is installed and this open-close lid 27 is closed during heating so that a high-frequency current flows sufficiently also through this open-close lid 27 as is the case with this embodiment, a turbulence of the electromagnetic field due to the window 17 can be suppressed to a minimum extent.

Also, this open-close lid 27 prevents the electromagnetic wave from leaking outside the cavity 12 and thereby a safe treatment can be secured.

### (B8) Embodiment 8

When the stereo resonator 11 is made rotatable relatively to the body 10 to be heated, a desired local portion can be heated intensively by heating the body 10 to be heated in an arbitrary direction or by rotating the stereo resonator 11, and thus the effect of medical treatment can be improved.

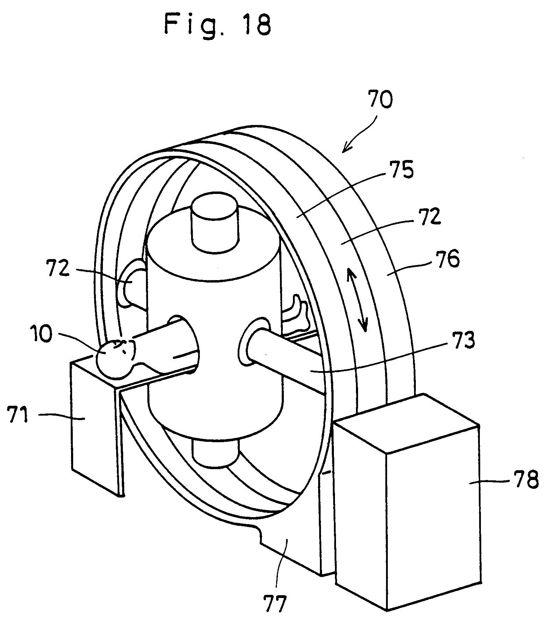

Fig. 18 shows one embodiment in this case. In Fig. 18, numeral 71 designates a putting table putting the body 10 to be heated, and numeral 70 designates a rotary mechanism. In the rotary mechanism 70, numeral 72 designates a circular rotary rack, which supports the stereo resonator 11 by support frames 73 and 74. Numerals 75 and 76 designate circular support racks, which rotatably supports the rotary rack 72. Numeral 77 designates a fixing leg, which fixes and supports the support racks 75 and 76. Numeral 78 is a rotation driving unit, which rotated the rotary rack 72.

In this configuration, when the rotary rack 72 is rotated by the rotation driving unit 78, the stereo resonator 11 is rotated, and thereby the body 10 to be heated can be heated in an arbitrary direction or can be heated while rotated.

### (C) Heating Experiments

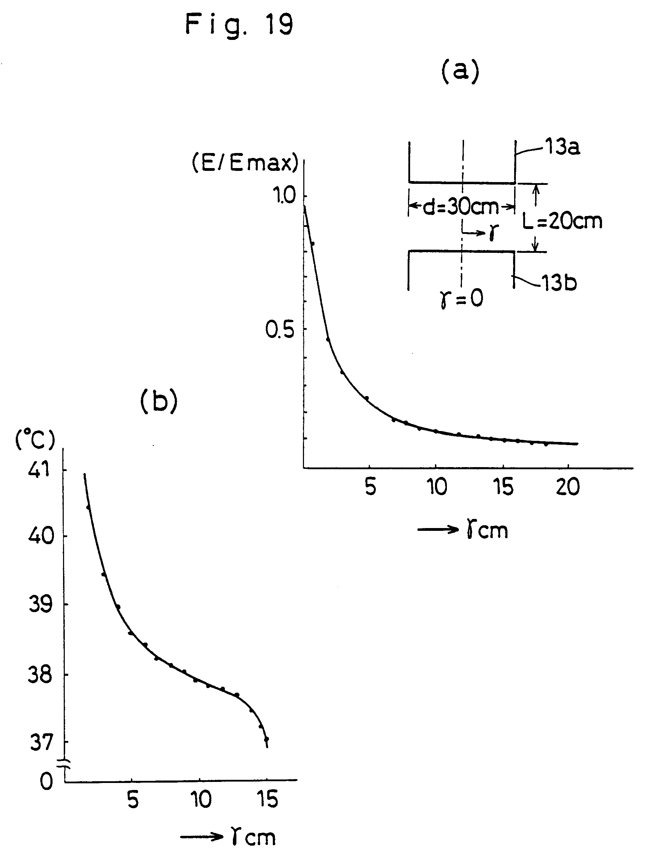

Fig. 19 shows the results of the heating experiments conducted by the locally heating apparatus of the present invention.

The diameter of the inner electrodes 13a and 13b is 30cm, the interval between the electrodes is 20cm, and the frequency is 140.75MHz. Fig. 19 (a) shows a distribution of electric field intensity, and the ordinate represents the electric field intensity and the abscissa represents the distance of the inner electrodes 13a and 13b from the center axis. Fig. 19 (b) shows a temperature distribution, and the ordinate represents the temperature and the abscissa represents the distance of the inner electrodes 13a and 13b from the center axis.

These results of the experiments show that the temperature becomes highest on the center axis of the inner electrodes 13a and 13b, and the electric field is converged and concentrated on the center axis. Also, in the heating experiments wherein fat and muscles are sandwiched in a superposed fashion, fat is hardly heated, and muscles are heated intensely, and thereby desirable results have been obtained for medical treatment.

### Utilizability in Industries

As described above, the stereo resonator for local heating and the locally heating apparatus in accordance with the present invention is useful for heating the body to be heated satisfactorily, and is specifically suitable for the heating treatment for perishing cancer cells.

## Claims

1. A cavity resonator for local heating of a body by applying high-frequency energy to said resonator, characterized by a cavity of cylindrical shape (12) in whose axis a conductive cylinder of smaller diameter is arranged to form an inner electrode (13), an electromagnetic field being generated in the gap between the face of the inner conductor (13) and the cavity by a source of microwave energy (30) coupled to the cavity by a probe (37).

2. A cavity resonator in accordance with claim 1, characterized by installing two inner electrodes (13a) and (13b) facing each other with an interval kept wherein the body (10) to be heated can be inserted.

3. A cavity resonator in accordance with claim 1 or claim 2, characterized in that the inner electrodes (13, 13a, 13b) are installed on the end surfaces (15, 15a, 15b) of the cylindrical cavity (12).

4. A cavity resonator in accordance with any one of claim 1 through claim 3, characterized by at least one entrance (14, 14a and 14b) for carrying the body (10) to be heated into or out of the cavity (12).

5. A cavity resonator in accordance with any one of claim 1 through claim 4, characterized in that at least one electrode surface of the inner electrode (13, 13a and 13b) has a structure generating a variable distribution of electric field.

6. A cavity resonator in accordance with any one of claim 1 through claim 5, characterized by at least one electric field distribution adjusting hole (16, 16a and 16b) for adjusting the electric field distribution formed by the inner electrode (13) in part of the conductor constituting the cavity (12).

7. A cavity resonator in accordance with any one of claim 1 through claim 6, characterized by at least one electric field adjusting member (22, 22a and 22b) for adjusting the electric field distribution formed by the inner electrode (13) in the cavity (12).

8. A cavity resonator in accordance with any one of claim 1 through claim 7, characterized in that part of the conductor constituting the cavity (12) is made movable.

9. A cavity resonator in accordance with any one of claim 1 through claim 8, characterized in that at least one of the inner electrodes (13, 13a and 13b) constituted with a conductor has a structure allowing to vary the position of the electrode surface thereof.

10. A cavity resonator in accordance with any one of claim 1 through claim 9, characterized by that at least one electrode surface of the inner electrodes (13, 13a and 13b) is covered with an insulating member (24).

11. A cavity resonator in accordance with any one of claim 1 through claim 10, characterized by that at least one of frequency adjusting members (21, 21a, 21b and 21c) for adjusting resoncance frequency is mounted in a manner capable of inserting in the cavity (12).

12. A locally heating apparatus characterized by comprising a cavity resonator 11 according to any one of claims 1 to 11 wherein a high-frequency power supply unit (30) is supplying this stereo resonator (11) for local heating with high-frequency energy.

13. A locally heating apparatus comprising a cavity resonator (11) to which high-frequency energy is applied for locally heating a body (10) to be heated by an electromagnetic field generated by said energy, characterised by a cavity (12) of cylindrical shape having a coaxial inner electrode (13) which is formed by hollowing part of the cavity (12) or by a separate conductor, the electromagnetic field being generated in the gap between the face of the inner electrode (13) and the cavity (12), frequency adjusting members (21, 21a, 21b and 21c) for adjusting resonance frequency mounted in a manner capable of being inserted in said cavity (12), a high-frequency power supply unit (30) for supplying this stereo resonator (11) with high-frequency energy, a resonant state detecting means (40) for detecting the resonant state of the cavity resonator (11), and a position controlling means (50) for adjusting the positions of said frequency adjusting members (21, 21a, 21b and 21c) to obtain the best resonant state in correspondence to the detected resonant state.

14. A locally heating apparatus in accordance with claim 12 or claim 13, characterized in that the frequency of high-frequency energy generated by the high-frequency power supply unit (30) is variable.

15. A locally heating apparatus in accordance with any one of claim 12 through claim 14, characterized by at least one shielding member (23, 23a and 23b) cutting off leakage of the electric field into portions other than the portion to be heated of the body (10) to be heated.

16. A locally heating apparatus in accordance with any one of claim 12 through claim 15, characterized by cooling members (25, 25a and 25b) in the inner electrodes (13, 13a and 13b), and by a cooling unit (50) supplying these cooling members (25, 25a and 25b) with cooling water.

17. A locally heating apparatus in accordance with any one of claim 12 through claim 16, characterized in that low-loss gap members (26, 26a and 26b) are inserted between at least one of the inner electrodes (13, 13a and 13b) and the body (10) to be heated.

18. A locally heating apparatus in accordance with any one of claim 12 through claim 17, characterized by at least one window (17) having an open-close lid (27) capable of opening and closing which is constituted with a conductor in part of the conductor constituting the cavity (12).

19. A locally heating apparatus in accordance with any one of claim 12 through claim 18, characterized by a putting table (71) putting the body (10) to be heated thereon and a rotary mechanism (70) rotating this putting table (71) relatively to the stereo resonator (11).

## Patentansprüche

1. Hohlraumresonator zur lokalen Heizung eines Körpers durch Anlegen einer Hochfrequenz-Energie an den Resonator,
**gekennzeichnet durch**
einen zylinderförmigen Hohlraum (12), in dessen Achse zur Bildung einer Innenelektrode (13) ein leitender Zylinder geringeren Durchmessers angeordnet ist, wobei ein elektromagnetisches Feld in dem Zwischenraum zwischen der Stirnseite des Innenleiters (13) und dem Hohlraum von einer Quelle (30) für Mikrowellenenergie erzeugt wird, die durch eine Sonde (37) in den Hohlraum eingekoppelt wird.

2. Hohlraumresonator nach Anspruch 1, gekennzeichnet durch die Anordnung von zwei Innenelektroden (13a,13b), die einander gegenüberstehen, wobei zwischen ihnen ein Zwischenraum verbleibt, in den der zu heizende Körper (10) einführbar ist.

3. Hohlraumresonator nach Anspruch 1 oder Anspruch 2, dadurch gekennzeichnet, daß die Innenelektroden (13,13a,13b) an den Stirnflächen (15,15a,15b) des zylindrischen Hohlraums (12) angeordnet sind.

4. Hohlraumresonator nach einem der Ansprüche 1 bis 3, gekennzeichnet durch mindestens einen Eingang (14,14a,14b) zum Tragen des zu heizenden Körpers (10) in oder aus dem Hohlraum (12).

5. Hohlraumresonator nach einem der Ansprüche 1 bis 4, dadurch gekennzeichnet, daß mindestens eine Elektrodenfläche der Innenelektrode (13,13a,13b) einen Aufbau aufweist, der eine veränderliche Verteilung eines elektrischen Feldes erzeugt.

6. Hohlraumresonator nach einem der Ansprüche 1 bis 5, gekennzeichnet durch mindestens ein Einstelloch der Verteilung des elektrischen Feldes (16,16a,16b) zum Einstellen der durch die Innenelektrode (13) in einem Teil des den Hohlraum (12) bildenden Leiters geformten Verteilung des elektrischen Feldes.

7. Hohlraumresonator nach einem der Ansprüche 1 bis 6, gekennzeichnet durch mindestens ein Einstellelement (22,22a,22b) des elektrischen Feldes zum Einstellen der durch die Innenelektrode (13) in dem Hohlraum (12) gebildeten Verteilung des elektrischen Feldes.

8. Hohlraumresonator nach einem der Ansprüche 1 bis 7, dadurch gekennzeichnet, daß ein Teil des den Hohlraum (12) bildenden Leiters beweglich gemacht ist.

9. Hohlraumsresonator nach einem der Ansprüche 1 bis 8, dadurch gekennzeichnet, daß mindestens eine der mit einem Leiter gebildeten Innenelektroden (13,13a,13b) einen Aufbau aufweist, der eine Veränderung der Position ihrer Elektrodenfläche ermöglicht.

10. Hohlraumresonator nach einem der Ansprüche 1 bis 9, dadurch gekennzeichnet, daß mindestens eine Elektrodenfläche der Innenelektroden (13,13a,13b) mit einem Isolierelement (24) bedeckt ist.

11. Hohlraumresonator nach einem der Ansprüche 1 bis 10, dadurch gekennzeichnet, daß mindestens eines von Frequenzeinstellelementen (21,21a,21b,21c) zum Einstellen einer Resonanzfrequenz in einer in den Hohlraum (12) einführungsfähigen Weise befestigt ist.

12. Vorrichtung zum lokalen Heizen, dadurch gekennzeichnet, daß ein Hohlraumresonator (11) nach einem der Ansprüche 1 bis 11 vorgesehen ist, wobei eine Versorgungseinheit (30) für Hochfrequenzleistung diesen Hohlraumresonator (11) zum lokalen Heizen mit Hochfrequenzenergie beliefert.

13. Vorrichtung zum lokalen Heizen mit einem Hohlraumresonator (11), dem Hochfrequenzenergie zum lokalen Heizen eines zu heizenden Körpers (10) durch ein von der Energie erzeugtes elektromagnetisches Feld zugeführt wird,
**gekennzeichnet durch**
einen zylinderförmigen Hohlraum (12) mit einer koaxialen Innenelektrode (13), die durch Aushöhlen eines Teils des Hohlraums (12) oder durch einen getrennten Leiter gebildet wird, wobei das elektromagnetische Feld in dem Zwischenraum zwischen der Stirnseite der Innenelektrode (13) und dem Hohlraum (12) erzeugt wird, Frequenzeinstellelemente (21,21a,21b,21c) zum Einstellen von Resonanzfrequenz, die in einer Weise befestigt sind, daß sie in den Hohlraum (12) ein-führbar sind, eine Hochfrequenzleistungsversorgungseinheit (30) zum Versorgen des Hohlraumresonators (11) mit Hochfrequenzenergie einer Resonanzfrequenzerfassungseinrichtung (40) zum Erfassen des Resonanzzustandes des Hohlraumresonators (11) und einer Positionssteuervorrichtung (50) zum Einstellen der Positionen der Frequenzeinstellelemente (21,21a,21b,21c), um den besten Resonanzzustand in Übereinstimmung mit dem erfaßten Resonanzzustand zu erhalten.

14. Vorrichtung zum lokalen Heizen nach Anspruch 12 oder Anspruch 13, dadurch gekennzeichnet, daß die Frequenz der von der Hochfrequenzleistungs-Versorgungseinheit (30) erzeugten Hochfrequenzenergie veränderlich ist.

15. Vorrichtung zum lokalen Heizen nach einem der Ansprüche 12 bis 14, gekennzeichnet durch mindestens ein Abschirmelement (23,23a,23b) zum Abschirmen der Streuung des elektrischen Feldes auf andere Bereiche als den zu heizenden Bereich des zu heizenden Körpers (10).

16. Vorrichtung zum lokalen Heizen nach einem der Ansprüche 12 bis 15, gekennzeichnet durch Kühlelemente (25,25a,25b) in den Innenelektroden (13,13a,13b), und durch eine Kühleinheit (50), die diese Kühlelemente (25,25a,25b) mit Kühlwasser versorgt.

17. Vorrichtung zum lokalen Heizen nach einem der Ansprüche 12 bis 16, dadurch gekennzeichnet, daß Zwischenelemente mit niedrigem Verlust (26,26a,26b) zwischen mindestens eine der Innenelektroden (13,13a,13b) und den aufzuheizenden Körper (10) eingefügt sind.

18. Vorrichtung zum lokalen Heizen nach einem der Ansprüche 12 bis 17, gekennzeichnet durch mindestens ein Fenster (17) mit einer zu öffnenden und zu schließenden Abdeckklappe (27), die als ein Leiter als Teil des den Hohlraum (12) bildenden Leiters ausgebildet ist.

19. Vorrichtung zum lokalen Heizen nach einem der Ansprüche 12 bis 18, gekennzeichnet durch einen Auflegetisch (71) zum Auflegen des zu heizenden Körpers (10) und durch einen Drehmechanismus (70), der diesen Auflegetisch (71) relativ zu dem Hohlraumresonator (11) dreht.

## Revendications

1. Résonateur à cavité pour chauffage local d'un corps par envoi d'une énergie haute fréquence vers ledit résonateur, caractérisé en ce qu'il comporte une cavité de forme cylindrique (12) dans l'axe de laquelle est agencé un cylindre conducteur plus petit pour former une électrode intérieure (13), un champ électromagnétique étant engendré dans l'espace existant entre la face du conducteur intérieur (13) et la cavité, par une source d'énergie micro-onde (30) reliée à la cavité par une sonde (37).

2. Résonateur à cavité selon la revendication 1, caractérisé par l'installation de deux électrodes intérieures (13a et 13b) situées en vis à vis l'une de l'autre, un espace étant maintenu entre elles, dans lequel peut être inséré le corps (10) destiné à être chauffé.

3. Résonateur à cavité selon la revendication 1 ou 2, caractérisé en ce que les électrodes intérieures (13, 13a, 13b) sont installées sur les surfaces d'extrémité (15, 15a, 15b) de la cavité cylindrique (12).

4. Résonateur à cavité selon l'une quelconque des revendications 1 à 3, caractérisé en ce qu'au moins une entrée (14, 14a, 14b) destinée au transport du corps (10) destiné à être chauffé vers l'intérieur de la cavité (12) ou vers l'extérieur de celle-ci.

5. Résonateur à cavité selon l'une quelconque des revendications 1 à 4, caractérisé en ce qu'au moins une surface d'électrode de l'électrode intérieure (13, 13a et 13b) a une structure engendrant une répartition variable du champ électrique.

6. Résonateur à cavité selon l'une quelconque des revendications 1 à 5, caractérisé en ce qu'il comporte au moins un trou (16, 16a et 16b) d'ajustement de la répartition du champ électrique pour ajuster la répartition du champ électrique formé par l'électrode intérieur (13) dans la partie du conducteur constituant la cavité (12).

7. Résonateur à cavité selon l'une quelconque des revendications 1 à 6, caractérisé en ce qu'au moins un élément d'ajustement du champ électrique (22, 22a et 22b) destiné à ajuster la répartition du champ électrique est formé par l'électrode intérieure (13) située dans la cavité (12).

8. Résonateur à cavité selon l'une quelconque des revendications 1 à 7, caractérisé en ce qu'une partie du conducteur constituant la cavité (12) est rendue mobile.

9. Résonateur à cavité selon l'une quelconque des revendications 1 à 8, caractérisé en ce qu'au moins une des électrodes intérieures (13, 13a et 13b) constituée d'un conducteur a une structure permettant de faire varier la position de sa surface d'électrode.

10. Résonateur à cavité selon l'une quelconque des revendications 1 à 9, caractérisé en ce qu'au moins une surface d'électrode des électrodes intérieures (13, 13a et 13b) est revêtue d'un élément isolant (24).

11. Résonateur à cavité selon l'une quelconque des revendications 1 à 10, caractérisé en ce qu'au moins un des éléments d'ajustement de fréquence (21, 21a, 21b et 21c) destiné à ajuster la fréquence de résonance est monté de manière à pouvoir être inséré dans la cavité (12).

12. Appareil de chauffage local caractérisé en ce qu'il comporte un résonateur à cavité (11) selon l'une quelconque des revendications 1 à 11, dans lequel un ensemble (30) d'alimentation électrique haute fréquence alimente ce résonateur stéréo (11) pour chauffage local à l'aide d'une énergie haute fréquence.

13. Appareil de chauffage local comportant un résonateur à cavité (11) auquel est appliquée une énergie haute fréquence pour chauffer localement un corps (10) destiné à être chauffé par un champ électromagnétique engendré par ladite énergie, caractérisé en ce qu'il comporte une cavité (12) de forme cylindrique ayant une électrode intérieure coaxiale (13) qui est formée en creusant une partie de la cavité (12) ou par un conducteur séparé, le champ électromagnétique étant engendré dans l'espace existant entre la face de l'électrode intérieure (13) et la cavité (12), des éléments d'ajustement de fréquence (21, 21a, 1b et 21c) pour ajuster la fréquence de résonance, montés de manière à pouvoir être insérés dans ladite cavité (12), un ensemble (30) d'alimentation électrique haute fréquence pour alimenter ce résonateur stéréo (11) en énergie haute fréquence, des moyens (40) de détection d'état résonnant pour détecter l'état résonnant du résonateur à cavité (11), et des moyens (50) de commande de position pour ajuster les positions desdits éléments d'ajustement de fréquence (21, 21a, 21b et 21c) pour obtenir le meilleur état résonnant en correspondance à l'état résonnant détecté.

14. Appareil de chauffage local selon la revendication 12 ou 13, caractérisé en ce que la fréquence de l'énergie haute fréquence engendrée par l'ensemble (30) d'alimentation électrique haute fréquence est variable.

15. Appareil de chauffage local selon l'une quelconque des revendications 12 à 14, caractérisé en ce qu'au moins un élément de blindage (23, 23a et 23b) arrête les fuites du champ électrique dans des parties autres que les parties destinées à être chauffées du corps (10) à chauffer.

16. Appareil de chauffage local selon l'une quelconque des revendications 12 à 15, caractérisé en ce qu'il comporte des éléments de refroidissement (25, 25a et 25b) situés dans les électrodes intérieures (13, 13a et 13b), et un ensemble de refroidissement (50) alimentant ces éléments de refroidissement (25, 25a et 25b) en eau de refroidissement.

17. Appareil de chauffage local selon l'une quelconque des revendications 12 à 16, caractérisé en ce que des éléments formant espace à faible perte (26, 26a et 26b) sont insérés entre au moins une des électrodes intérieure (13, 13a et 13b) et le corps (10) destiné à être chauffé.

18. Appareil de chauffage local selon l'une quelconque des revendications 12 à 17, caractérisé en ce qu'il comporte au moins une fenêtre (17) ayant un couvercle (27) d'ouverture/fermeture capable d'ouvrir et de fermer, qui est constitué à l'aide d'un conducteur pris dans une partie du conducteur constituant la cavité (12).

19. Appareil de chauffage local selon l'une quelconque des revendications 12 à 18, caractérisé en ce qu'il comporte une table de présentation (71) présentant le corps (10) destiné à être chauffé et un mécanisme rotatif (70) mettant en rotation cette table de présentation (71) par rapport au résonateur stéréo (11).
